# NEW EUROPEAN PATENT SPECIFICATION

(11) **EP 1 003 532 B2**
(45) Date of publication and mention of the opposition decision: **05.11.2008**
(45) Mention of the grant of the patent: 07.11.2001
(21) Application number: 98939584.3
(22) Date of filing: 26.06.1998
(51) Int. Cl.: A61K 35/74, A23C 9/123, A23L 1/03

(54) **LACTOBACILLI TO INCREASE ABSORPTION OF MINERALS BY INTESTINAL CELLS**
LACTOBACILLI ZUR ERHOEHUNG DER MINERALIENABSORPTION DURCH DARMZELLEN
LACTOBACILLI POUR AUGMENTER L'ABSORPTION DE MINERAUX PAR DES CELLULES INTESTINALES

(30) Priority: 05.07.1997 EP 97111380
(43) Date of publication of application: 31.05.2000
(73) Proprietor: SOCIETE DES PRODUITS NESTLE S.A., 1800 Vevey (CH)
(72) Inventor: BRASSART, Dominique, F-53940 Saint Berthevin (FR); VEY, Elisabeth, CH-1196 Gland (CH)
(74) Representative: Straus, Alexander
(86) International application number: PCT/EP1998/004036
(87) International publication number: WO 1999/002170

(56) References cited:
- EP-A- 0 457 539
- EP-A- 0 577 904
- DR. J. LJ. RASIC ET AL.: "FERMENTED FRESH MILK PRODUCTS, VOLUME 1: YOGHOURT, SCIENTIFIC GROUNDS, TECHNOLOGY, MANUFACTURE AND PREPARATIONS." 1978 , DR. J. LJ. RASIC ET AL. , BEOGRAD, YU XP002052238 cited in the application see page 114, paragraph 1 - paragraph 2 see page 115; table 31
- T. YAESHIMA: "BENEFITS OF BIFIDOBACTERIA TO HUMAN HEALTH." BULLETIN OF THE INTERNATIONAL DAIRY FEDERATION, no. 313, 1996, pages 36-42, XP002052237 cited in the application
- PATENT ABSTRACTS OF JAPAN vol. 098, no. 011, 30 September 1998 & JP 10 158178 A (YAKULT HONSHA CO LTD), 16 June 1998
- Taishi, O. et al.; J. Nutr. Sci. Vitaminol., 1994, 40:613-616
- Wolf, B.W. et al.; Microbial Ecology in Health and Disease, 1995, 8:41-50
- Le Ferrec, E. et al.; ATLA, 2001, 29:649-668; Report of the 46th ECVAM workshop
- Tuomola, E.M. et al.; Int. J. of Food Microbiology, 1998, 41:45-51

## Description

This invention relates to the use of probiotic Lactobacilli micro-organisms in the preparation of an enteral composition for facilitating or increasing the absorption, by mammals, of minerals from the general diet.

Minerals are key elements in major physiological processes. Calcium is, for example, of vital importance for the formation of bones and teeth, muscle contraction and the synthesis of hormones. Calcium is also an essential secondary messenger in most cell activation phenomena.

Minerals, of which the diet is the primary source, are assimilated by the body by crossing the intestinal mucosa so as to then pass into the blood stream. The degree of assimilation (or of absorption) of minerals by the body in fact depends both on their solubility in the intestinal medium and on the capacity of the intestinal cells to assimilate them and to transfer them into the blood stream (R. Wasserman et al., In Mineral Absorption in the Monogastric GI Trac. Advances in Experimental Medicine and Biology, 249, 45-65. Plenum Press, N.Y., 1989).

The location, the efficiency and the mechanisms of calcium absorption all along the intestine have been studied in rats and chickens for many years (Bronner F., J. Nutr., 122, 641-643, 1992: Schachter D., Am. J. Physiol., 196, 357-362. 1959). For obvious ethical and technical reasons, such studies have been limited in man (Hylander E. et al., Scand. J. Gastroenterol., 25, 705, 1990) and onlv a few *in vitro* studies have been undertaken (Elsherydah A. et al., Gastroenterology, 109, 876, 1995; Feher J.J., Am. J. Physiol., 244, C303, 1983: Feher J.J., Cell Calcium, 10, 189, 1989).

One of the most widely studied aspects of mineral absorption is the bioavailability of the minerals depending on the composition of the daily diet (Bronner F., J. Nutr., 123, 797, 1993). However, many minerals which are highly bioavailable are also instable and are unsuitable for use in the diet. Further, merely supplementing the diet with greater amounts of minerals often has a negative effect on the organo-leptic properties of the diet.

A possible solution to the problem is to facilitate or improve the absorption of minerals from the diet. However there have been few studies on methods of facilitating or increasing the absorption of minerals from the diet and the results have not heen consistent.

Rasic *et al*. have reported that the minerals contained in dairy products are assimilated better when these products are fermented. This effect is attributed to the presence of acids in the fermented dairy products (XP002052238: *In* Fermented Fresh Milk Product, volume 1, p114-115, 1978).

More recently, Yaeshima *et al.* have also shown an increase in the absorption of calcium in rats from a diet of calcium-fortified whey when a combination of oligosaccharides and *Bifidobacteria* is consumed (XP002052237: Bulletin of the International Dairy Fermentation, No. 313, 1996).

However, Kot *et al*. Have reported that *Lactobacillus acidophilus* naturally internalizes Fe²⁺, and oxidizes it to Fe³⁺; which is an insoluble form which is more difficult to assimilate (J. Agric. Food Chem., 43, 1276-1282, 1995).

Therefore there remains a need for a means of facilitating or increasing the absorption of minerals present in the diet.

Accordingly, this invention provides the use of probiotic *lactobacilli* in accordance with claim 1.

It has been surprisingly found, by use of an *in vitro* model, that probiotic *lactobacilli* are able to directly facilitate or improve the absorption of minerals, i.e. calcium, magnesium and/or zinc, by human intestinal cells. Without wishing to be bound by theory, this is thought to be linked to induction of acidification of the microenvironment around the intestinal cells and the bacteria in contact with the intestinal cells. Both the bacteria and the intestinal cells may participate in the induction of acidification. This localized acidification might thus play an active role in the solubilization of minerals, and therefore in the capacity of the body to assimilate them.

Embodiments of the invention are now described, by way of example only, with reference to the drawings in which:
Figure 1 represents the basal absorption of calcium by Caco-2 intestinal cells in the absence of *lactobacilli*;
Figure 2 represents the influence of about 6.7x10⁷ cfu/ml of various strains of *lactobacilli* on the absorption of calcium by Caco-2 intestinal cells;
Figure 3 represents the influence of about 3.4x10⁸ cfu/ml of various strains of *lactobacilli* on the absorption of calcium by Caco-2 intestinal cells.

The invention relates to the use of probiotic lactobacilli for the preparation of a nutritional composition for enteral administration to facilitate or improve the absorption of minerals present in a daily diet. The minerals are calcium, magnesium and/or zinc. The ingestion of probiotic *lactobacilli* increases the bioavailability of the minerals, that is to say makes the minerals, which are often not very soluble in the intestine, more accessible to the intestinal cells.

Any food-grade, probiotic *lactobacilli* strain may be used. For example, the following probiotic *lactobacilli* may be used: *Lactobacillus acidophilus*, *Lactobacillus crispatus, Lactobacillus amylovorous, Lactobacillus gallinarum, Lactobacillus gasseri* and *Lactobacillus johnsonii; Lactobacillus paracasei; Lactobacillus reuterii; Lactobacillus brevis; Lactobacillus fermentum; Lactobacillus plantarum; Lactobacillus casei* especially *L. casei subsp. casei* and *L. casei subsp. rhamnosus; Lactobacillus delbruckii* especially *L. delbruckii subsp*. *lactis, L. delbruckii subsp. helveticus* and *L. delbruckii subsp. bulgaricus;* and *Leuconostoc mesenteroides* especially *L. mesenteroides subsp. cremoris,* for example (Bergey's Manual of Systematic Bacteriology, vol. 2, 1986; Fujisawa et al., Int. Syst. Bact, 42, 487-491, 1992).

The probiotic *lactobacilli* may be capable of adhering to intestinal cells but need not be. However, the probiotic *lactobacilli* are preferably such that at least 50 bacteria, in particular at least 80 bacteria, are capable of adhering *in vitro* to 100 intestinal cells. To select such an adherent type of bacteria, a culture of bacteria may be spread on a confluent culture of an immortalized line of epithelial cells of the intestine (EP 0802257), the confluent culture washed, and the number of bacteria adhering to the villosities of the line measured.

Some strains are in fact capable of adhering to human intestinal cells, of excluding pathogenic bacteria which are on human intestinal cells, and/or of acting on the human immune system by allowing it to react more strongly to external aggression (immunomodulation capacity), for example by increasing the phagocytosis capacity of the granulocytes derived from human blood (J. of Dairy Science, 78, 491-197, 1995; immunomodulation capacity of the La-1 strain which was deposited by Nestec SA with the treaty of Budapest in the Collection Nationale de Culture de Microorganisme (CNCM), 25 rue docteur Roux, 75724 Paris, 30 June 1992, where it was attributed the deposit number CNCM I-1225). This strain is described in EP 0577904

By way of example, it is possible to use the probiotic strain *Lactobacillus acidophilus* CNCM I-1225. This strain was recently reclassified among the *Lactobacillus johnsonii* bacteria, subsequent to the new taxonomy, proposed by Fujisawa *et al*., which is now authoritative in the field of taxonomy of acidophilic lactobacilli (Int. J. Syst. Bact., 42, 487-791, 1992). Other probiotic bacteria are also available, such as those described in EP0199535 (Gorbach *et al*.). US5296221 (Mitsuoka *et al*.), US556785 (Institut Pasteur) or US5591428 (Probi AB), for example.

The nutritional compositions preferably comprise a sufficient quantity of live probiotic *lactobacilli* for a facilitated absorption of minerals by the intestinal cells, for example at least 10⁶ cfu/ml, in particular 10⁷-10¹¹ cfu/ml, preferably 10⁸-10¹¹ cfu/ml ("cfu" means "colony forming unit").

The nutritional composition may also contain other bacteria as desired; for example other probiotic bacteria.

The nutritional composition may also include a suitable protein source; for example an animal or plant protein source. Suitable protein sources are milk proteins, soy proteins, rice proteins, wheat proteins, sorghum proteins, and the like. The proteins may be in intact or hydrolyzed form.

The nutritional composition may also include a suitable carbohydrate source; for example sucrose, fructose, glucose, maltodextrin, and the like.

The nutritional composition may also include a suitable lipid source; for example a suitable animal or plant lipid source. Suitable lipid sources include milk fats. sunflower oil, rapeseed oil, olive oil, safflower oil, and the like.

The nutritional composition may also be fortified with minerals and vitamins. It is especially preferred to fortify the nutritional composition with calcium.

The nutritional compositions may be prepared in the form of food compositions intended for human or animal consumption. Suitable food compositions may be provided in the form of liquids, powders, and solids.

The nutritional composition may be fermented to obtain a sufficient quantity of probiotic *lactobacilli*. Fermented compositions based on milk are thus particularly suitable. The term milk applies not only to animal milks but also to what is commonly called a vegetable milk, that is to say an extract of treated or untreated plant materials such as legumes (soya, chick pea. lentil and the like) or oilseeds (rape, soya, sesame, cotton and the like), which extract contains proteins in solution or in colloidal suspension, which are coagulable by chemical action, by acid fermentation and/or by heat. It has been possible to subject these vegetable milks to heat treatments similar to those for animal milks. It has also been possible to subject them to treatments which are specific to them, such as decolorization, deodorization. and treatments for suppressing undesirable tastes. Finally, the word milk also designates mixtures of animal milks and of plant milks.

It is also possible to add, mix or coat the nutritional composition, during its preparation, with an appropriate quantity of a culture of probiotic *lactobacilli* in liquid, concentrated, dry or encapsulated form, according to need.

It has been found that the microencapsulation of the probiotic *lactobacilli* has therapeutic advantages. First, microencapsulation significantly increases the survival of the probiotic *lactobacilli* and therefore the number of live probiotic *lactobacilli* which arrive in the intestine. Even more importantly, the probiotic *lactobacilli* are gradually released into the intestine, which permits prolonged action of the probiotic *lactobacilli* on the absorption of minerals by the intestinal cells.

Preferably, to encapsulate probiotic *lactobacilli*, the probiotic *lactobacilli* are freeze-dried or spray-dried (EP0818529), and they are incorporated into a gel consisting, for example, of a solidified fatty acid, a sodium alginate, polymerized hydroxypropylmethylcellulose or polymerized polyvinylpyrrolidone. To this effect, the teaching given in FR2.443.247 is incorporated by reference.

The nutritional compositions need not contain carbohydrates necessary for active fermentation by probiotic *lactobacilli* in the intestinal medium. On the contrary, the facilitated absorption of minerals is independent of the fermentative activity of the probiotic *lactobacilli*, but rather appears to result from the direct contact between the *lactobacilli* and the intestinal cells. This is thought to induce acidification of the microenvironment and therefore a better solubilization of the minerals.

However, it may be desirable to provide for renewal or specific multiplication of the probiotic *lactobacilli* in the intestinal medium so as to prolong the effect of facilitated absorption of the minerals. This may be achieved by adding fibres which facilitate the specific multiplication of probiotic *lactobacilli* in the intestinal medium to the nutritional composition, These fibres are soluble and fermentable.

These fibres may be selected from, for example, plant pectins, chito-. fructo-, gentio-, galacto-, isomalto-. manno- or xylo-oligosaccharides or oligosaccharides from soya, for example (Playne et al., Bulletin of the IDF 313. Group B42, Annual Session of September 95, Vienna).

The preferred pectins are polymers of α-1,4-D-galacturonic acid having a molecular weight of the order of 10 to 400 kDa, which can be purified from carrots or tomatoes, for example (JP60164432). The preferred galactooligosaccharides comprise a saccharide portion consisting of 2 to 5 repeating units of structure [-α-D-Glu-(1→4)-β-D-Gal-(1→6)-] (Yakult Honsa Co.. Japan). The preferred fructo-oligosaccharides are inulin-oligofructoses extracted from chicory which may comprise, for example, 1-9 repeating units of structure [-β-D-Fru-(1→2)-β-D-Fru-(1→2)-] (WO94/12541; Raffinerie Tirlemontoise S.A., Belgium), or oligosaccharides synthesized from sucrose units which may comprise, for example, a saccharide portion consisting of 2 to 9 repeating units of structure [-α-D-Glu-(1→2)-β-D-Fru-(1→2)-] (Meiji Seika Kasiha Co., Japan). The preferred malto-oligosaccharides comprise a saccharide portion consisting of 2 to 7 repeating units of structure [-α-D-Gal-(1→4)-] (Nihon Shokuhin Kako Co., Japan). The preferred isomaltoses comprise a saccharide portion consisting of 2 to 6 repeating units of structure [-α-D-Glu-(1→6)-] (Showa Sangyo Co., Japan). The preferred gentio-oligosaccharides comprise a saccharide portion consisting of 2 to 5 repeating units of structure [-β-D-Glu-(1→6)-] (Nihon Shokuhin Kako Co., Japan). Finally, the preferred xylo-oligosaccharides comprise a saccharide portion consisting of 2 to 9 repeating units of structure [-β-xyl-(1→4)-] (Suntory Co., Japan), for example.

The quantity of fibres in the nutritional composition depends on their capacity to promote the development of probiotic *lactobacilli.* As a general rule, the nutritional composition may contain from I to 50% of such fibres (by weight relative to the dry matter). The concentration of probiotic *lactobacilli* may be at least 10³ CFU of probiotic *lactobacilli* per g of fibres, preferably 10⁴ to 10⁷ CFU/g of fibres.

Another advantage provided by the fibres consists in the fact that the intestinal transit is retarded by the fibres. This is particularly the case if the quantity of fibres is large, that is to say of the order of 20-50% relative to the weight of the composition. The probiotic *lactobacilli* being gradually eliminated by the action of the intestinal transit, it is possible, in this manner, to prolong the beneficial action of the probiotic *lactobacilli* on the absorption of minerals by the intestine.

The nutritional compositions may be in the form of any suitable enterally administered food. For example, the nutritional composition may take the form of a fermented milk (EP0577904). an infant (EP0827697), a fromage frais (PCT/EP97/06947), a ripened cheese, an ice cream (WO 98/09535), a biscuit filled with a cream (EP704164; EP666031), a dry sausage and/or a pâté (EP689769).

The nutritional compositions may also be in a form suitable for people who cannot tolerate dairy products. These nutritional compositions will not contain allergenic milk derivatives. For example, for children who are allergic to milk proteins, the nutritional composition may be formulated to contain hypoallergenic milk derivatives. These milk derivatives may be in accordance with European directive 96/4/EC which states that in a hypoallergenic milk, the allergenic proteins should be immunologically at least 100 times less detectable than in a nonhydrolysed milk (Off. J. Europ. Comm., NoL49/12, annex point 5.a. 1996; Fritsché et al., Int. Arch. Aller. and Appl. Imm., 93, 289-293, 1990).

The nutritional compositions are particularly suitable for the treatment or prophylaxis of people having mineral deficiencies, or to compensate for physiological deficiencies due to a diet low in minerals, or to satisfy major physiological requirements for minerals in children, pregnant women, women who are breastfeeding and the elderly.

This invention is now further described by means of specific examples.
The percentages are given by weight unless otherwise indicated. These examples are given by way of illustration only and do not in any manner constitute a limitation of the invention.

### Example 1

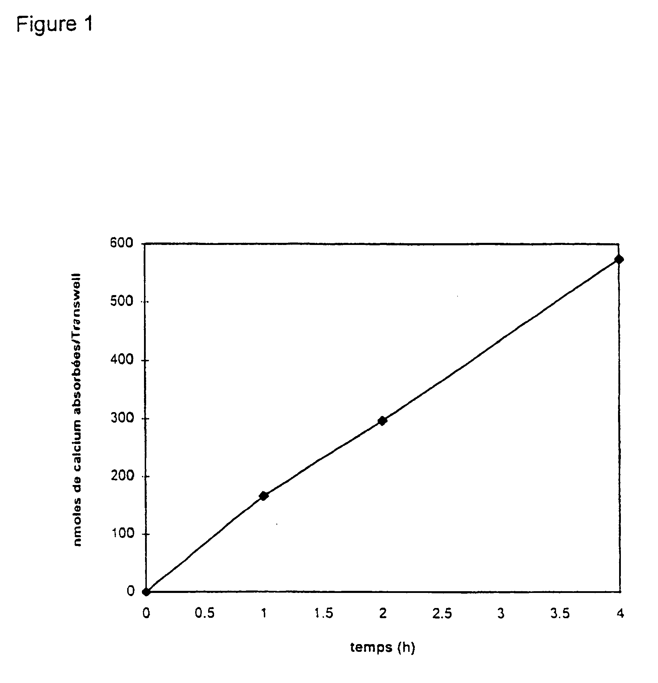

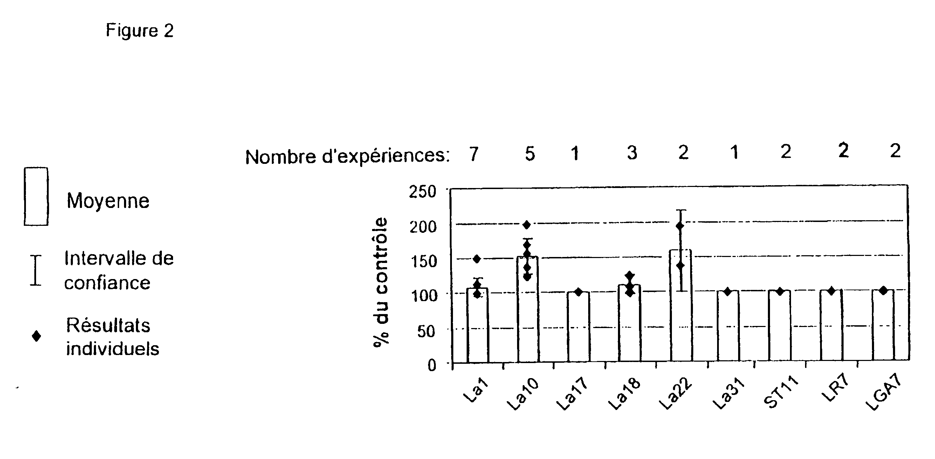

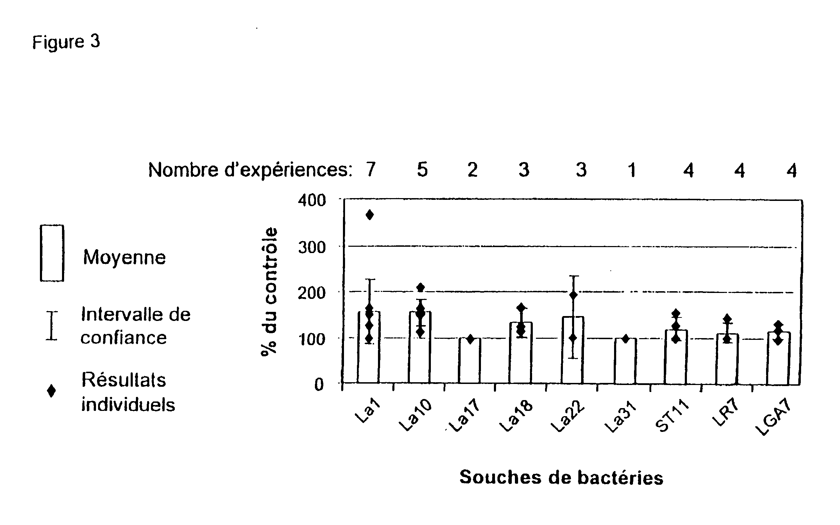

- Materials: ⁴⁵CaCl₂ is obtained from Amersham. Lucifer yellow from Sigma, collagen I from Centrix Pharmaceuticals, PBS, HEPES and the components of the cell culture medium from Gibco, and the culture supports from Falcon.
- Cell culture: the human cell line Caco-2, isolated from a colon
adenocarcinoma, is obtained from American Type Culture Collection (passage 41). The cells are placed in culture in an amount of 4x10⁴ cells/cm² in DMEM containing 4.5 g/l of glucose. 20% heat-inactivated foetal calf serum, 1 mg/ml of fungizone, 100 U/ml of penicillin/streptomycin, 200 µg/ml of gentamycin and 1% of nonessential amino acids. The cells are regularly tripsinized and placed in culture again at 1:20. The cells used in the calcium transport experiments are placed in culture at 1x10⁵ cells/cm² in permeable inserts previously coated with a layer of collagen I at 50 µg/ml. In all cases, the cells are maintained in a 10% CO₂/90% air incubator at 37°C, and the medium is replaced every two days.
- Viability of the Caco-2 cells: in order to exclude the possibility that the potentiation of the absorption of calcium by the intestinal cells in the presence of *lactobacilli* is due to cellular damage, a portion of each sample serving for the assay of calcium was used for an assay of the hexosaminidase activity (Landegren *et al*., J. Immunol. Method. 67. 379-378. 1984). This colorimetric test makes it possible to quantify cell lysis and/or death by measuring the hexosaminidase activity released into the supernatant from the cytosol of damaged cells. The results show that in all the experiments, the hexosaminidase activity is equivalent in the presence of *lactobacilli*.
- Permeability of the cellular lawn: the integrity of the lawn formed by the Caco-2 cells at the end of their growth and of their differentiation is evaluated by measuring the transepithelial electrical resistance (TEER) using a voltmeter/ohmmeter Millicell-ERS. The calcium absorption experiments are carried out when this resistance reaches at least 700 ohm x cm². The permeability of the cellular lawn during the calcium absorption experiments is evaluated by measuring the level of diffusion (in %) of Lucifer yellow, a molecule which does not cross the cell membrane.
- Transport of calcium: the Caco-2 cells are cultured on inserts for 3 to 5 weeks. On the day of the experiment, the cellular lawn is washed twice in PBS and then the bottom compartment of the insert incorporating the serosa (basolateral pole of the cells) receives 2.5 ml of carrier buffer (140 mM NaCl, 5.8 mM KCl.
0.34 mM NaH₂PO₄, 0.44 mM KH₂PO₄, 0.8 mM MgSO₄, 20 mM HEPES. 4 mM glutamine, 25 mM glucose, pH 7.4) supplemented with 2.5 mM CaCl₂, whereas the top compartment of the insert incorporating the intestinal lumen (apical pole of the cells) receives 1.5 ml of carrier buffer supplemented with 10 mM CaCl₂ and trace amounts of ⁴⁵CaCl₂ and Lucifer yellow. The inserts are then placed at 37°C and 50 µl of sample in the bottom and top compartments are removed at regular intervals.
The radioactivity contained in these samples is evaluated by liquid scintillation counting and makes it possible to extrapolate on the quantity of cold CaCl₂ absorbed. The basal transport of calcium is expressed as nmol of calcium transported to the bottom compartment of the insert. The diffusion of Lucifer yellow detected by spectrofluorometry in the bottom compartment is expressed in % of the quantity introduced into the top compartment.
- Influence of the *lactobacilli*: the strains *Lactobacillus johnsonii* La1 (CNCMI-1225). La17, La22, La31; *Lactobacillus acidophilus* La10, La18, La31;
*Lactobacillus bulgaricus* Lfi5, YL8; *Lactobacillus paracasei* ST11; *Lactobacillus gasseri* LGA7; *Lactobacillus reuteri* LR7 and *Streptococcus thermophilus* Sfi20, YS4 (Nestec collection. Lausanne. Switzerland) are placed in culture under anaerobic conditions in MRS broth for *Lactobacillus* or M17 for *Streptococcus* for two times 24 h. washed in PBS and resuspended in carrier buffer before being introduced into the top compartment of the inserts. The Caco-2:bacteria ratio is then about 1:100 according to the tests (6.7x10⁷ or 3.4x10⁸ cfu/ml in the top compartment of the inserts, for the tests presented in Figures 2 and 3). The absorption of calcium is evaluated according to the protocol mentioned above.
- Results of the basal transport of calcium: a calcium gradient was established in the inserts by introducing 2.5 mM CaCl₂ into the bottom compartment, which corresponds to the normal human plasma concentration, and arbitrarily 10 mM CaCl₂ into the top compartment, which would correspond to the calcium content of a food diet. As shown by the results of a representative experiment illustrated by Figure 1, the basal absorption of calcium by the Caco-2 cells increases with time to reach up to 600 nmol/insert, comprising about 3x10⁶ cells, after 4 h. As a check for the integrity of the cellular lawn during the experiment, the diffusion of Lucifer yellow was measured and proved to be less than 2%.
- Measurement of the influence of *lactobacilli*: in Figures 2 and 3, the absorption of calcium by the Caco-2 cells is increased significantly in the presence of the adherent *Lactobacillus johnsonii* strains La1 and La22, in the presence of the non-adherent La10 and La18 *Lactobacillus acidophilus* strains, and in the presence of the *L. paracasei* (ST11), *L*. *gasseri* (LGA7) and *L. reuterii* (LR7) strains.
The capacity of the bacteria to adhere to the intestinal cells therefore does not appear to correlate directly with their capacity to increase the absorption of calcium by these same cells. In all these experiments, the diffusion of Lucifer yellow is modulated in a similar manner but remains negligible.
A decrease in pH in the top compartment of the inserts is also observed when the Caco-2 cells are in the presence of *lactobacilli*, regardless of the strain. except with the Sfi20 strain (Table 1). There is therefore no correlation between the increase in the absorption of calcium and this decrease in pH. However certain bacterial strains capable of increasing the absorption of calcium are not capable of acidifying the experimental medium in the absence of Caco-2. This means that the acidification in the presence of Caco-2 and of bacteria requires a collaboration between the two types of organisms and could be due to the Caco-2 cells.

**Table 1:**

| Influence of *lactobacilli* on the pH of the experimental medium in the absence or in the presence of Caco-2 cells | | | |
|---|---|---|---|
| Bacteria | Number of tests | pH without Caco-2 | pH with Caco-2 |
| None | 4 | 7 +/- 0 | 7 +/- 0 |
| La1 | 3 | 6.75 +/- 0.3 | 3.75 +/- 0.3 |
| La10 | 3 | 4.65 +/- 0.3 | 4.15 +/- 0.3 |
| La17 | 2 | 7 +/- 0 | 3.5 +/- 0.7 |
| La18 | 2 | 7 +/- 0 | 3.5 +/- 0.5 |
| La22 | 2 | 7 +/- 0 | 3.25 +/- 0.35 |
| La29 | 2 | 4.25 +/- 0.35 | 3.5 +/- 0 |
| La31 | 2 | 7 +/- 0 | 3.75 +/- 0.35 |
| Sfi20 | 1 | 7 | 7 |
| YS4 | 1 | 5 | 4 |
| Lfi5 | 1 | 4 | 3 |
| YL8 | 1 | 4 | 3 |

### Example 2

Tests similar to those carried out in Example 1 were carried out to determine the influence of *lactobacilli* on the absorption of calcium by the intestinal cells in the presence of labelled inulin (³H-inulin, Amersham; tracer prebiotic fibre). The results confirm that probiotic *lactobacilli* increase *in vitro* the absorption of minerals by the intestinal cells.

### Example 3

Tests similar to those carried out in Example 1 were carried out in order to determine the influence of *lactobacilli* on the absorption of magnesium, iron and zinc by the intestinal cells. The results confirm that probiotic *lactobacilli* increase *in vitro* the absorption of minerals by the intestinal cells.

### Example 4 Encapsulation of lactic acid bacteria

In a 100 l tank, 80 l of culture medium having the following composition, in %, are prepared:

| | |
|---|---|
| Yeast extract | 0.25% |
| Trypticase | 1.00% |
| Phytone | 0.50% |
| Glucose | 1.50% |
| L-cysteine HCl | 0.05% |
| K₂HPO₄ | 0.25% |
| ZnSO₄ | 0.025% |
| FeCl₃ | Trace |
| Water | Balance to 100% |

Inoculation is carried out with 1 l of a 20 h culture of *Lactobacillus johnsonii* Lal (CNCM I-1225). The medium is incubated for 12 h at 30°C. The culture broth is centrifuged and 240 g of cells are recovered. They are diluted in 250 ml of skimmed milk supplemented with 7% lactose. The mixture is frozen using liquid nitrogen. The freeze-drying is performed at 40°C overnight. A 5% dispersion of the powder obtained is prepared in hydrogenated vegetable fat having a melting point of 42°C and liquefied at 45°C. The dispersion is injected at 45°C under a pressure of 4 bar, at the same time as liquid nitrogen, in an amount of 1 part of dispersion for 5 parts of nitrogen, at the top of a vertical cylinder 1.5 m in diameter and 10 m high. A container is placed at the bottom of the cylinder, which contains liquid nitrogen in which the microbeads containing the bacteria whose diameter varies between 0.1 and 0.5 m are collected. The microbeads are then placed in a fluidized bed and an alcoholic solution containing 8% zein is sprayed over the bed, in a quantity such that the zein layer formed around the microbeads represents 5% of their weight.

The microbeads are then incorporated into a food composition intended to facilitate the absorption of minerals by the intestinal cells.

### Example 5

A concentrated base for ice cream is prepared by mixing at 60-65°C for 20 min about 11% of lactic fat, 8.8% of milk solids (solids-not-fat), 25% sucrose. 5% of glucose syrup and 0.6% of Emulstab® SE30. The base is homogenized at 72-75°C and at 210 bar (2 stages at 210/50 bar), it is pasteurized at 85°C for 22 sec (APV pasteurizer. France, Evreux, 400 l/h), it is cooled to 4°C and 40% of milk acidified by *Lactobacillus johnsonii La-1* (5x10⁸ cfu/ml) and *Bifidobacterium longum Bil6* (3x10⁸ cfu/ml) strains is added thereto. The composition of this concentrated base is presented in the table below.

| Ingredients | Composition (kg) | Fat (%) | Solids-not-fat(%) | Sucrose (%) | Dry extract (%) |
|---|---|---|---|---|---|
| Cream (35%) | 31.43 | 11.00 | 1.57 | | 12.57 |
| Skimmed milk powder | 7.60 | | 7.30 | | 7.30 |
| Sucrose | 36.77 | | | 25.00 | 25.00 |
| Glucose syrup | 5.27 | | | | 5.00 |
| Emulstab® SE30 | 0.67 | | | | 0.63 |
| Water | 18.26 | | | | |
| Total: cream base | 100.00 | 11.00 | 8.87 | 25.00 | 50.50 |
| Cream base (60%) | 60.00 | 6.60 | 5.32 | 15.00 | 30.30 |
| Acidified milk (40%) | 40.00 | 1.40 | 4.68 | - | 6.08 |
| Total: cream base + acidified milk | 100.00 | 8.00 | 10.00 | 15.00 | 36.38 |

Alter maturation of the cream for 12 h at 5°C, it is frozen to an overrun of 95% by volume (Crepaco freezer. France, Evreux; 160 1 of product/h).

A wafer dough is prepared which contains 10% fructo-oligosaccharide Raftilose® L30 (Raffinerie Tirlemontoise S.A., BE), according to the recipe reproduced in the table below. After baking, the wafer is conventionally formed into a cone. After cooling, the inside of the cones is spray-coated with a fatty film and then the cones are filled with the whipped ice cream described above. For an 11.5 g wafer cone, 130 ml of whipped ice cream (about 65 g) and 5 g of chocolate (spraying over the cream) are thus used.

| Ingredient | Weight (g) | Supplier |
|---|---|---|
| Ordinary wheat flour 55 | 52 | |
| Starch | 0.2 | |
| Fructo-oligosaccharide | 10 | Raffinerie Tirlemontoise S.A., |
| Raftilose® L30 | | BE |
| Sugar | 27.8 | |
| Fat | 8 | |
| Emulsifier | 1.5 | |
| Salt | 0.5 | |
| Total: wafer recipe | 100 | |

1.1 g of fibres and about 10⁸ cfu/g of *lactobacilli* are thus provided per ice cream cornet. The fibres, by promoting the specific development of *lactobacilli* in the intestinal tract, thus promote the assimilation of minerals.

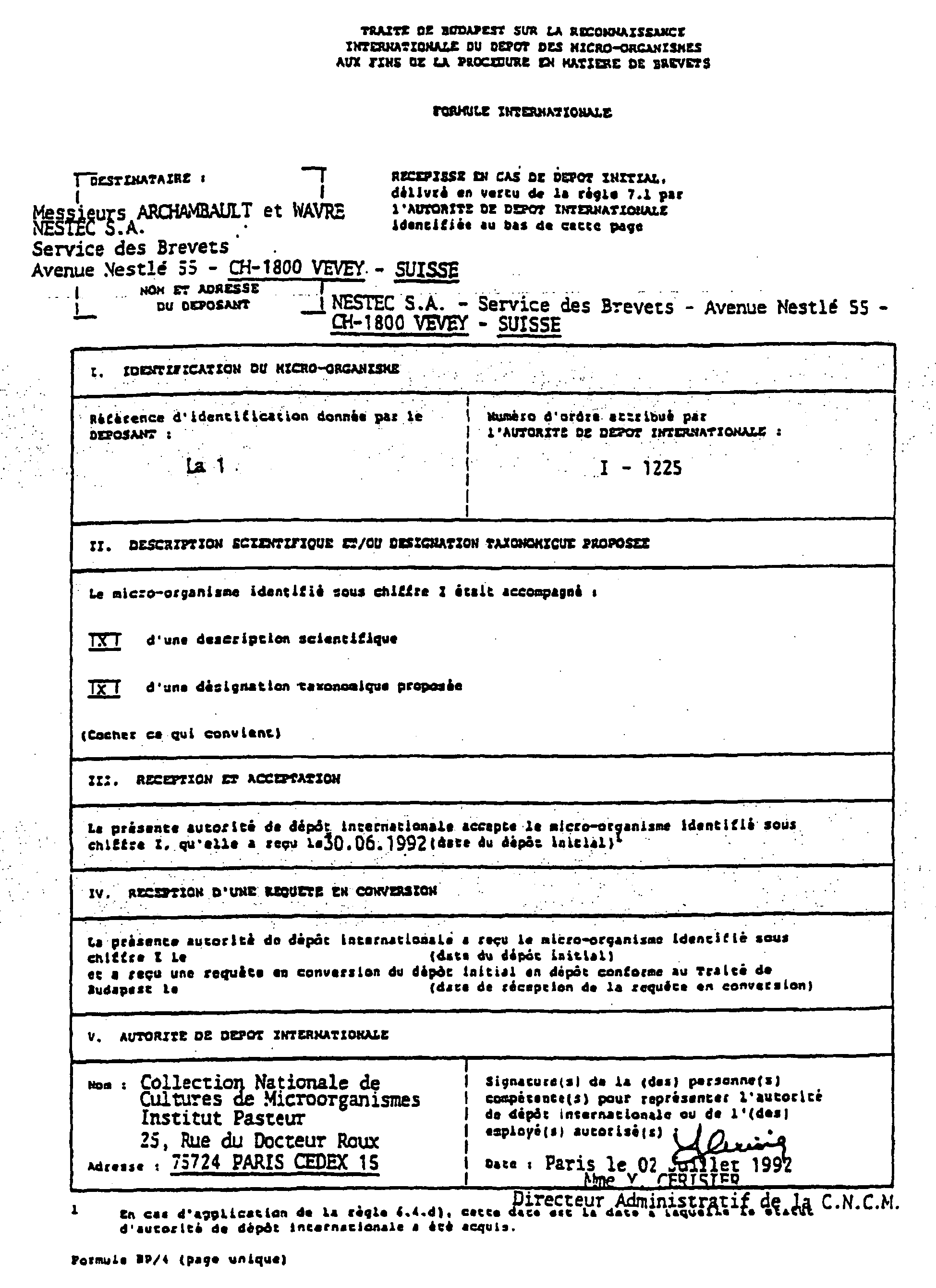

## Claims

1. Use of probiotic *lactobacilli* in the preparation of an enteral nutritional composition for the treatment or prophylaxis of deficiencies in calcium, magnesium and/or zinc.

2. Use according to claim 1 in which the *lactobacilli* is the *Lactobacillus* bacteria which is capable of adhering to intestinal cells.

3. Use according to claim 2 in which the *lactobacilli* is the *Lactobacillus johnsonii* CNCM I-1225 strain.

4. Use according to claim 1 in which the enteral nutritional composition contains 10⁷ to 10¹¹ cfu of *lactobacilli.*

5. Use according to claim 1 in which the enteral nutritional composition contains milk proteins.

6. Use according to claim 5 in which the enteral nutritional composition is an infant formula comprising hypo-allergenic milk protein hydrolysates.

7. Use according to claim 1 in which the enteral nutritional composition further comprises prebiotic fibres.

## Patentansprüche

1. Verwendung von probiotischen Milchsäurebakterien bei der Herstellung einer enteralen Ernährungszusammensetzung für die Behandlung oder Vorbeugung von Calcium-, Magnesium- und/oder Zink-Mangelzuständen.

2. Verwendung nach Anspruch 1, bei der die Milchsäurebakterien solche von den Milchsäurebakterien sind, die in der Lage sind, an Darmzellen zu adhärieren.

3. Verwendung nach Anspruch 2, bei der die Milchsäurebakterien der *Lactobacillus johnsonii* CNCM I-1225 Stamm ist.

4. Verwendung nach Anspruch 1, bei der die enterale Ernährungszusammensetzung 10⁷ bis 10¹¹ KBE von Milchsäurebakterien beinhaltet.

5. Verwendung nach Anspruch 1, bei der die enterale Ernährungszusammensetzung Milchproteine beinhaltet.

6. Verwendung nach Anspruch 5, bei der die enterale Ernährungszusammensetzung eine Säuglingsnahrungszubereitung ist, die hypoallergene Milchproteinhydrolysate umfasst.

7. Verwendung nach Anspruch 1, bei der die enterale Ernährungszusammensetzung weiterhin präbiotische Fasern umfasst.

## Revendications

1. Utilisation de lactobacilles probiotiques dans la préparation d'une composition nutritionnelle entérale pour le traitement ou la prophylaxie de déficiences en calcium, magnésium et zinc.

2. Utilisation suivant la revendication 1, dans laquelle les lactobacilles sont des bactéries du genre Lactobacillus capables d'adhérer aux cellules intestinales.

3. Utilisation suivant la revendication 2, dans laquelle les lactobacilles consistent en la souche Lactobacillus johnsonii CNCM I-1225.

4. Utilisation suivant la revendication 1, dans laquelle la composition nutritionnelle entérale contient 10⁷ à 10¹¹ ufc de lactobacilles.

5. Utilisation suivant la revendication 1, dans laquelle la composition nutritionnelle entérale contient des protéines du lait.

6. Utilisation suivant la revendication 5, dans laquelle la composition nutritionnelle entérale est une formulation pour nourrissons comprenant des hydrolysats de protéines laitières hypoallergéniques.

7. Utilisation suivant la revendication 1, dans laquelle la composition nutritionnelle entérale comprend en outre des fibres prébiotiques.
